# EUROPEAN PATENT SPECIFICATION

(11) **EP 0 431 868 B1**
(45) Date of publication and mention of the grant of the patent: **21.09.1994**
(21) Application number: 90313102.7
(22) Date of filing: 03.12.1990
(51) Int. Cl.: C07D 249/20, C08K 5/3475, G02B 1/04

(54) **Bis(benzotriazolyl)benzophenones and their use as UV absorbers**
Bis(benzetriazolyl)benzophenone und ihre Verwendung als UV-Absorber
Bis(benzotriazolyl)benzophénones et leur utilisation comme absorbants UV

(30) Priority: 04.12.1989 US 445277; 22.05.1990 US 527019
(43) Date of publication of application: 12.06.1991
(73) Proprietor: IOPTEX RESEARCH INC., Irwindale, California 91706-2094 (US)
(72) Inventor: Gupta, Amitava, San Marino, California (US)
(74) Representative: Gilholm, Stephen Philip

(56) References cited:
- US-A- 4 260 768
- US-A- 4 716 234
- US-A- 4 745 194
- Chem. Abs. vol. 107, 1987, 178130h (JP-A-62-172071)

## Description

This invention relates to UV absorbers, to polymer compositions containing such absorbers and to articles made from such UV absorber-containing polymer compositions. More particularly the invention relates to a novel ultraviolet absorber, to acrylic polymers having incorporated therein a UV absorbing amount of the UV absorber, to intraocular lenses and contact lenses formed from said acrylic polymers and to eyeglasses formed from a polycarbonate material which contains a UV absorbing amount of the UV absorber.

United States Patent No.4,745,194 discloses ultraviolet light absorbing compounds having two different chromophores in the same molecule, particularly the benzotriazole chromophore and either the dihydroxybenzophenone or dihydroxyacetophenone chromophore. More particularly it relates to 3,5-[di(2H-benzotriazole-2-yl)]-2,4-dihydroxybenzophenone and to 3,5-[di(2H-benzotriazole-2-yl)]-2,4-dihydroxyacetophenone. It does not disclose 3,5[Bis (2H-benzotriazole-2-yl)] 2,4,4'-trihydroxybenzophenone (BTHB).

Chem. Abs., vol. 107, 1987 abstract 178130h (JP-A-62,172,071) discloses weather-resistant coatings containing, eg. 2,4,4'-trihydroxy benzophenone added as an auxochrome. The benzophenone is not substituted by any groups apart from hydroxy groups. In particular the benzophenone is not substituted by triazole groups.

United States Patent No. 4,260,768 discloses copolymerisable ultraviolet light absorber compounds, and more particularly 2-(2H-benzotriazol-2-yl)-4-alkylphenol acrylic acid ester compounds. There is no disclosure of benzophenone based UV-absorbers.

United States Patent No. 4,716,234 discloses compositions comprising benzotriazole-based UV absorbing compounds. There is no disclosure of benzophenone based UV-absorbers.

In accordance with the present invention there is provided a compound of the formula (I) 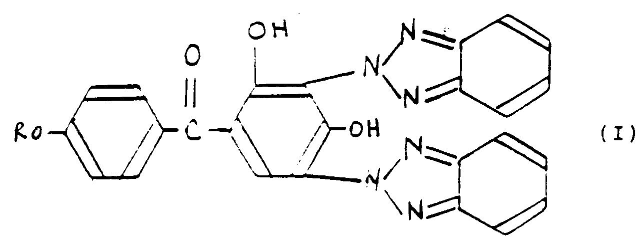
wherein R is hydrogen or -CO-CR¹=CH₂, wherein R¹ is hydrogen or methyl.

Preferred compounds of Formula (I) are:
3,5[Bis(2H-benzotriazol-2yl)] 2,4,4'-trihydroxybenzophenone (BTHB) and
3,5[Bis(2H-benzotriazol-2-yl)]2,4-dihydroxy 4'-acryloyl oxybenzophenone (BTAB).

The compounds of the present invention may be produced by conventional techniques. For example, the triazole component may be first produced by conventional diazotising techniques followed by reaction with the appropriate benzophenone. The resultant ditriazole can then be esterified, for example, by acylation with an appropriate acrylic component to produce a polymerisable triazole.

These compounds are effective for absorbing UV light in the range of about 200nm to about 450nm particularly in the range of 240nm to 420nm.

The present invention also provides a polymer composition comprising a UV absorbing amount of the compound of Formula I herein.

The polymer composition may be a polymer per se comprising residues derived from a acrylically unsaturated monomer eg. methyl methacrylate and a UV absorbing amount of residues derived from the compound of Formula I herein.

The UV absorber-containing acrylic polymers of the present invention are believed to have the formula (II) 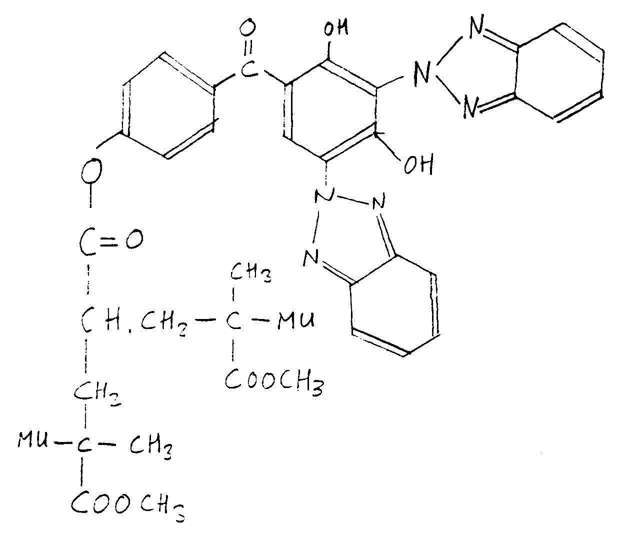
wherein MU is the monomer unit.

Preferred polymers are those derived from methylmethacrylate. Other polymers may comprise residues derived from other acrylically unsaturated compounds such as acrylates or methacrylates for example hydroxyethylmethacrylate.

The amount of compound of Formula I required to be a effective UV absorber will generally be greater than 0.1% by weight of the total composition. Normally the amount of UV absorber need not exceed 5% by weight of the composition. Thus the UV absorber of Formula I may aptly be included in the polymer or polymer composition in an amount of from 0.1% to 5% by weight based on the weight of the polymer composition. Suitably 0.1% to about 2% by weight of the UV absorber may be incorporated. About 0.15% by weight is a preferred amount.

In accordance with a further aspect of the invention there is provided an article comprising a polymer or polymer composition in accordance with the invention.

According to an embodiment of this further aspect of the present invention, an intraocular lens (IOL) is provided which comprises a polymer comprising residues derived from methylmethacrylate having incorporated an UV absorbing amount of residues of UV absorber of formula (I) herein described wherein R is a -CO-CR¹=CH₂ group and R¹ is as defined hereinabove.

According to a further embodiment of the present invention, a contact lens is provided which comprises a polymer having residues derived from methylmethacrylate and UV absorbing amount of residues of UV absorber of formula (I) wherein R is a -CO-CR¹=CH₂ group and R¹ is as defined hereinabove.

Such intraocular or contact lens may comprise polymer material as herein described having from about 10ppm to about 500ppm of the UV absorber of formula (I) incorporated per part of polymer. From about 100ppm to about 250ppm is a preferred amount.

The intraocular lenses according to the present invention, may have haptics, eg two secured or affixed thereto or integral therewith and such haptics may have any of the known configurations or modifications thereof.

Both intraocular lenses (IOLs) and the contact lenses of the present invention may be produced by any of the conventional procedures and techniques per se known in the art. See, for example, US Patent No. 4102567; US Patent No. 4208364; US Patent No. 4158030; US Patent No. 3408429; US Patent No. 3361858; European Patent Application No. 0328246; European Patent Application No.0333348; Technical and Economic Effects of Contact Lens Production Methods by P. Cordrey, Optical World, 1972, Sept./Oct., pages 13 to 20; Intraocular Lens Implantation Design, E.S.P. Ford from Intraocular Lens Implantation edited by E.S. Rosen.

According to a further embodiment of the present invention, eyeglasses are produced having lens made of a polycarbonate material suitable for eyeglass lenses which has incorporated therein a UV absorbing amount of a compound of Formula (I). Aptly the lens material will have from about 10ppm to about 500ppm of the UV absorber of Formula (I) per part of polycarbonate material. From about 100ppm to about 250ppm is a preferred amount.

For the IOLs, the contact lenses and the eyeglasses of the present invention the amount of the novel UV absorber which is incorporated can also be expressed as a weight percentage based on the weight of the polymer or polycarbonate material. Preferably, the weight percentage of the novel UV absorber incorporated is from about 0.1% to about 5% preferably from about 0.1% to about 2%. About 0.15% by weight is a preferred amount.

The invention will be illustrated by reference to the accompanying drawings in which:

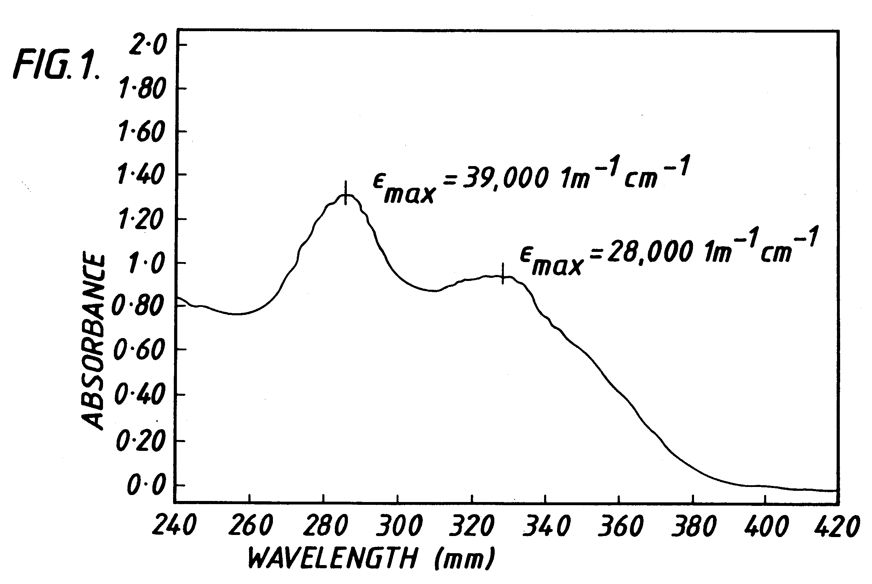

Figure 1 shows an IR spectrum for BTAB.

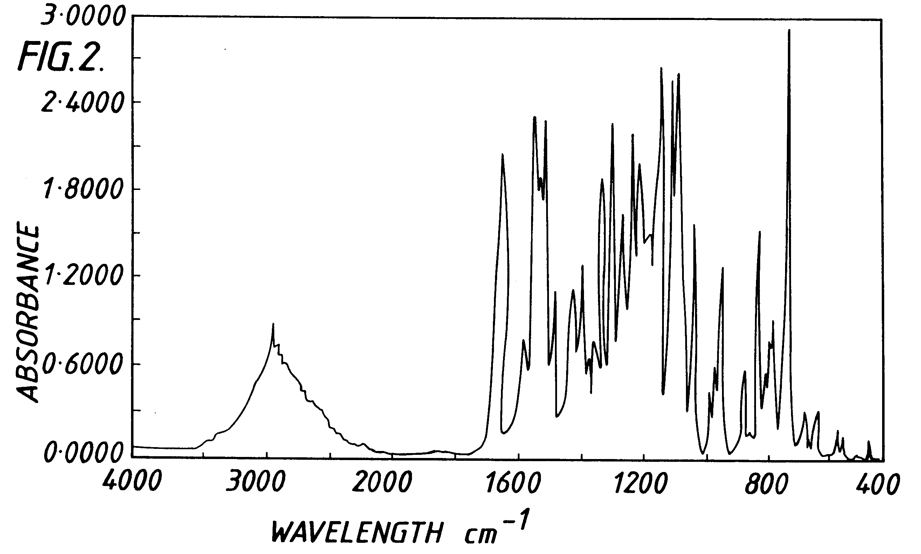

Figure 2 shows a UV spectrum for BTAB.

The following examples are given to further illustrate the present invention.

### Example 1

### 3,5-[Di(2H-benzotriazole-2-yl)] 2,4,4'-trihydroxy benzophenone (BTHB)

A solution of o-nitroaniline (11.1g, 0.08M) in concentrated hydrochloric acid (30cc) was diluted with 30cc of water and diazotised with a solution of sodium nitrite (5.69g, 0.08M) in water (20cc) at 0°-5°C. The cold solution of o-nitrobenzene diazonium chloride was added over a period of one hour with stirring to a solution of 2,4,4' trihydroxybenzophene (9.2g, 0.04M), sodium hydroxide (7.29g, 0.18M), and sodium bicarbonate (22g, 0.2M) in water (300ml) at 0°-5°C.

The red diazo compound was collected, washed to neutral with water, dissolved in 170cc aqueous sodium hydroxide (13.6g), and reductively cyclised with Zn dust (13.6g) at room temperature in 24 hours.

Subsequently, the suspension was filtered, the residue washed with 10% aqueous NaOH; the filtrate was combined with this extract and acidified with sulfuric acid while keeping the temperature below 10°C.

Precipitated crude ditriazole was collected by filtration, air-dried, extracted with ethanol to yield 9.25g of product.

### Example 2

Acryloyl chloride (2g, 0.02M) was added dropwise to a stirred solution of 3,5-[Di(2H-benzotriazole-2-yl)2,4,4'-trihydroxybenzophenone (4.6g, 0.01M) in 150cc of 0.4 molar aqueous sodium hydroxide solution at 10°C. The reaction mixture was stirred at room temperature for 30 minutes and the precipitate collected by filtration.

The crude product was recrystallised from acetone-water.

| | |
|---|---|
| Yield 2.8g | 90% pure by TLC |

The product was 3,5-[Bis(2H-benzotriazole-2-yl)2,4,-dihydroxy, 4'-acryloyloxy-benzophenone (BTAB).

### Example 3

Copolymerisation of the product of Example 1 with methylmethacrylate was carried out at 65°C in sealed tubes using USP-245 as an initiator.

This material has a polymerisation reactivity comparable to substituted styrenes, and hence can be incorporated into a wide variety of polymers by addition copolymerisation or grafting. Examples of polymers in which it can be incorporated are:
By copolymerisation: polyolefins, polyacrylates, polymethacrylates, PVC, polystyrene and derivatised polystyrenes.
By grafting: polycarbonates, polyesters, polyacrylates and polymethacrylates.

### Example 4

An intraocular lens was formed by first forming a UV absorber containing polymer and thereafter cutting out discs from the formed polymer sheet.

The polymer was formed by mixing the following components:

| | |
|---|---|
| Methylmethacrylate | 99.7 parts per hundred |
| BTAB | 0.15 pph |
| OT100 (mould release agent)¹ | 100 parts per million |
| USP245 (free radical initiator)² | 0.15 pph |

| | |
|---|---|
| 1 Sodium dioctyl sulphsuccinate [Cyanamicid] | |
| 2 2,5-Dimethyl-2,5-di(2-ethylhexanoyl peroxy) hexane. | |

After mixing the components were degassed bubbling argon gas therethrough and then placing the mixture in a mould.

The mixture was then heated at 55°C for 10 hours after which the temperature was raised to 90°C for a further 24 hours.

After polymerisation the formed sheet was post cured for 8 hours at 100°C and then cooled to room temperature.

Disc blanks were cut from the formed sheet and machined to produce intraocular lenses.

## Claims (Claims for the following Contracting State(s): DE, GB, FR, IT, NL, SE, LI, CH, BE, AT, LU, DK)

1. A compound of the formula (I): 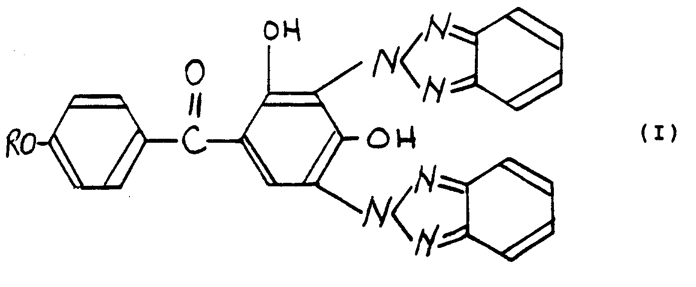 wherein R is hydrogen or -CO-CR¹=CH₂, wherein R¹ is hydrogen or methyl.

2. 3,5[Bis(2H-benzotriazol-2-yl)]2,4,4'-trihydroxy benzophenone.

3. 3,5[Bis(2H-benzotriazol-2-yl)]2,4-dihydroxy 4'-acryloyloxy benzophenone.

4. A polymer composition comprising a UV absorbing amount of the compound of Formula 1 defined in claim 1.

5. A polymer comprising residues derived from methyl methacrylate and residues derived from the compound defined in claim 1 wherein R is -CO-CR¹=CH₂ and R¹ is as defined in claim 1.

6. A polymer or polymer composition as claimed in claim 4 or claim 5 wherein the amount of compound of
Formula (I) is from 0.1 to 5% by weight of the polymer or composition.

7. An article produced from a polymer or polymer as claimed in any one of claims 4 to 6.

8. An intraocular lens or a contact lens comprising a polymer produced using a method as claimed in claim 5.

9. An intraocular lens as claimed in claim 8 having at least one haptic attached thereto or formed integrally therewith.

10. A lens according to claim 9 having two haptics.

11. An article as claimed in claim 7 in the form of eyeglasses having lenses made from a polymer composition comprising a polycarbonate resin having incorporated therein a UV absorbing amount of the compound of Formula (I).

## Claims (Claims for the following Contracting State(s): ES)

1. A method of providing a UV absorbing compound comprising forming a compound of the formula (I): 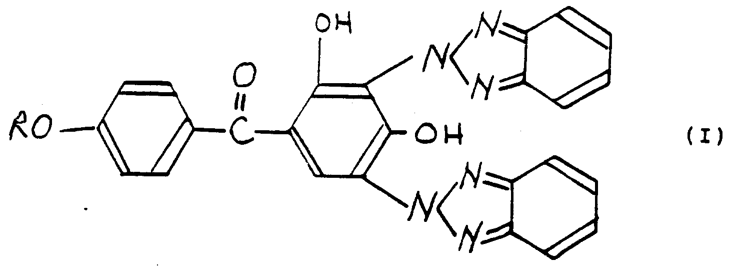 wherein R is hydrogen or -CO-CR¹=CH₂ wherein R¹ is hydrogen or methyl.

2. A method for providing a UV absorbing compound comprising forming 3,5[Bis(2H-benzotriazol-2-yl)]2,4,4'-trihydroxy benzophenone.

3. A method of providing a UV absorbing compound comprising forming 3,5[Bis(2H-benzotriazol-2-yl)]2,4-dihydroxy 4'-acryloyloxy benzophenone.

4. A method of forming a polymer composition comprising a UV absorbing compound, comprising incorporating into a polymer a UV absorbing amount of the compound of Formula 1 defined in claim 1.

5. A method of forming a polymer comprising UV absorbing residues, comprising incorporating into a polymer residues derived from methyl methacrylate and residues derived from the compound defined in claim 1 wherein R is -CO-CR¹=CH₂ and R¹ is as defined in claim 1.

6. A method of providing a UV absorbing compound comprising forming a method as claim in claim 4 or claim 5 wherein the amount of the compound of
Formula (I) is from 0.1 to 5% by weight of the polymer or composition.

7. An article produced using a method as claimed in any one of claims 4 to 6.

8. An intraocular lens or a contact lens comprising a polymer produced using a method as claimed in claim 5.

9. An intraocular lens as claimed in claim 8 having at least one haptic attached thereto or formed integrally therewith.

10. A lens according to claim 9 having two haptics.

11. An article as claimed in claim 7 in the form of eyeglasses having lenses made from a polymer composition comprising a polycarbonate resin having incorporated therein a UV absorbing amount of the compound of Formula (I).

## Patentansprüche (Patentansprüche für folgende(n) Vertragsstaat(en): DE, GB, FR, IT, NL, SE, LI, CH, BE, AT, LU, DK)

1. Verbindung der Formel (I): 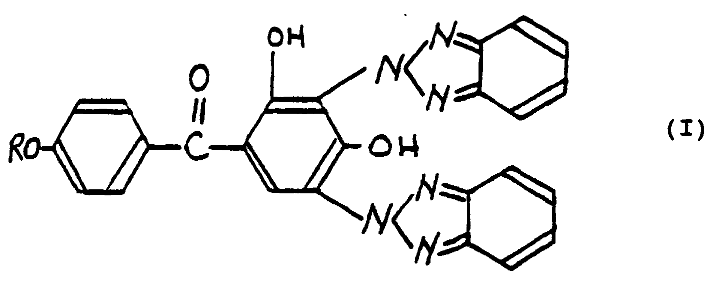 worin R Wasserstoff oder -CO.CR¹=CH₂ ist, worin R¹ Wasserstoff oder Methyl ist.

2. 3,5-[Bis(2H-benzotriazol-2-yl)]-2,4,4'-trihydroxybenzophenon.

3. 3,5-[Bis(2H-benzotriazol-2-yl)]-2,4-dihydroxy-4'-acryloyloxybenzophenon.

4. Polymerzusammensetzung, die eine UV-absorbierende Menge der Verbindung der in Anspruch 1 definierten Formel 1 umfaßt.

5. Polymer, das von Methacrylsäuremethylester abgeleitete Reste und von der in Anspruch 1 definierten Verbindung, worin R -CO.CR¹=CH₂ ist und R¹ wie in Anspruch 1 definiert ist, abgeleitete Reste umfaßt.

6. Polymer oder Polymerzusammensetzung wie in Anspruch 4 oder Anspruch 5 beansprucht, worin die Menge der Verbindung der Formel (1) von 0,1 bis 5 Gew.-% des Polymers oder der Zusammensetzung beträgt.

7. Gegenstand, der aus einem Polymer oder aus einem in den Ansprüchen 4 bis 6 beanspruchten Polymer hergestellt ist.

8. Intraokuläre Linse oder Kontaktlinse, die ein mittels eines in Anspruch 5 beanspruchten Verfahrens hergestellten Polymer umfaßt.

9. Intraokuläre Linse wie in Anspruch 8 beansprucht mit wenigstens einem daran befestigten oder daran einstückig angeformten Haltebügel.

10. Linse gemäß Anspruch 9 mit zwei Haltebügeln.

11. Gegenstand wie in Anspruch 7 beansprucht in Form einer Brille mit Linsen, die aus einer Polymerzusammensetzung hergestellt sind, die ein Polycarbonatharz mit einer darin eingearbeiteten, UV-absorbierenden Menge der Verbindung der Formel (I) umfaßt.

## Patentansprüche (Patentansprüche für folgende(n) Vertragsstaat(en): ES)

1. Verfahren zum Bereitstellen einer UV-absorbierenden Verbindung, umfassend das Bilden einer Verbindung dar Formel (I): 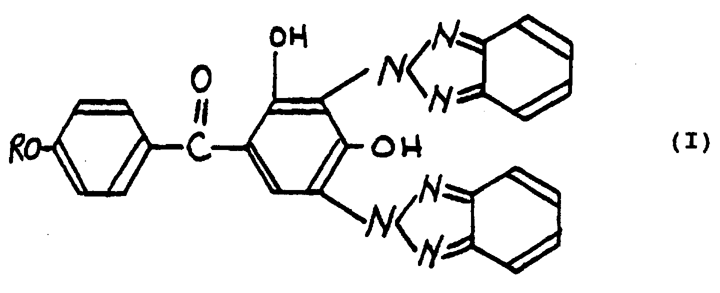 worin R Wasserstoff oder -CO.CR¹=CH₂ ist, worin R¹ Wasserstoff oder Methyl ist.

2. Verfahren zum Bereitstellen einer UV-absorbierenden Verbindung, umfassend das Bilden von 3,5-[Bis(2H-benzotriazol-2-yl)]-2,4,4'-trihydroxybenzophenon.

3. Verfahren zum Bereitstellen einer UV-absorbierenden Verbindung, umfassend das Bilden von 3,5-[Bis(2H-benzotriazol-2-yl)]-2,4-dihydroxy-4'-acryloyloxybenzophenon.

4. Verfahren zum Bilden einer eine UV-absorbierende Verbindung umfassenden Polymerzusammensetzung, welches das Einarbeiten einer UV-absorbierenden Menge der Verbindung der in Anspruch 1 definierten Formel 1 in ein Polymer umfaßt.

5. Verfahren zum Bilden eines UV-absorbierende Reste umfassenden Polymers, welches das Einarbeiten von Methacrylsäuremethylester abgeleiteter Reste und von der in Anspruch 1 definierten Verbindung, worin R -CO.CR¹=CH₂ ist und R¹ wie in Anspruch 1 definiert ist, abgeleiteter Reste in ein Polper umfaßt.

6. Verfahren zum Bereitstellen einer UV-absorbierenden Verbindung, umfassend das Ausführen eines in Anspruch 4 oder Anspruch 5 beanspruchten Verfahrens, worin die Menge der Verbindung der Formel (1) von 0,1 bis 5 Gew.-% des Polymers oder der Zusammensetzung beträgt.

7. Gegenstand, der mittels eines in einem der Ansprüche 4 bis 6 beanspruchten Verfahrens hergestellt ist.

8. Intraokuläre Linse oder Kontaktlinse, die ein mittels eines in Anspruch 5 beanspruchten Verfahrens hergestelltes Polymer umfaßt.

9. Intraokuläre Linse wie in Anspruch 8 beansprucht mit wenigstens einem daran befestigten oder daran einstückig angeformten Haltebügel.

10. Linse gemäß Anspruch 9 mit zwei Haltebügeln.

11. Gegenstand wie in Anspruch 7 beansprucht in Form einer Brille mit Linsen, die aus einer Polymerzusammensetzung hergestellt sind, die ein Polycarbonatharz mit einer darin eingearbeiteten, UV-absorbierenden Menge der Verbindung der Formel (I) umfaßt.

## Revendications (Revendications pour l'(les) Etat(s) contractant(s) suivant(s): DE, GB, FR, IT, NL, SE, LI, CH, BE, AT, LU, DK)

1. Composé de formule : 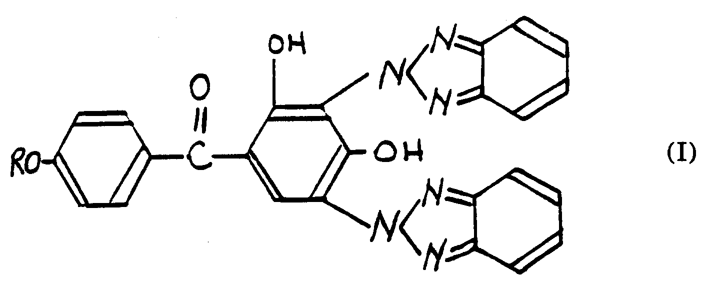 dans laquelle R est un atome d'hydrogène ou un groupe -CO.CR¹=CH₂ dans lequel R¹ est un atome d'hydrogène ou un groupe méthyle.

2. 3,5-[Bis-(2H-benzotriazol-2-yl)]-2,4,4'-trihydroxy benzophénone.

3. 3,5-[Bis-(2H-benzotriazol-2-yl)]-2,4-dihydroxy-4'-acryloyloxy benzophénone.

4. Composition de polymère comprenant une quantité absorbant les UV du composé de formule (I) défini dans la revendication 1.

5. Polymère comprenant des résidus dérivés de méthacrylate de méthyle et des résidus dérivés du composé défini dans la revendication 1, dans lequel R est un groupe -CO.CR¹=CH₂ et R¹ est tel que défini dans la revendication 1.

6. Polymère ou composition de polymère suivant la revendication 4 ou la revendication 5, où la quantité de composé de formule (I) est comprise entre 0,1 et 5% en poids du polymère ou de la composition de polymère.

7. Article produit à partir d'un polymère ou d'une composition de polymère suivant l'une quelconque des revendications 4 à 6.

8. Lentille intraoculaire ou lentille de contact comprenant un polymère produit selon un procédé suivant la revendication 5.

9. Lentille intraoculaire suivant la revendication 8, ayant au moins un haptique attaché à celle-ci ou formé de façon intégrale avec de celle-ci.

10. Lentille suivant la revendicaton 9, ayant deux haptiques.

11. Article suivant la revendication 7, sous la forme de vertes d'oeil ayant des lentilles faites à partir d'une composition de polymère comprenant une résine polycarbonate dans laquelle est incorporée une quantité absorbant les UV du composé de formule (I).

## Revendications (Revendications pour l'(les) Etat(s) contractant(s) suivant(s): ES)

1. Procédé fournissant un composé absorbant les UV, comprenant la formation d'un composé de formule (I) : 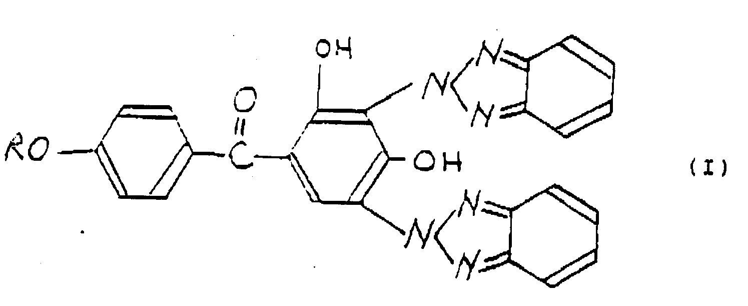 dans laquelle R est un atome d'hydrogène ou un groupe -CO.CR¹=CH₂ , dans lequel R¹ est un atome d'hydrogène ou un groupe méthyle.

2. Procédé fournissant un composé absorbant les UV, comprenant la formation de 3,5[bis(2H-benzotriazol-2-yl)]-2,4,4'-trihydroxybenzophénone.

3. Procédé fournissant un composé absorbant les UV, comprenant la formation de 3,5[bis(2H-benzotriazol-2-yl)]2,4-dihydroxy-4'-acryloyloxy benzophénone.

4. Procédé pour la formation d'une composition de polymère comprenant un composé absorbant les UV, lequel procédé comprend l'incorporation dans un polymère d'une quantité du composé absorbant les UV de formule (I) tel que défini dans la revendication 1.

5. Procédé pour la formation d'un polymère comprenant des résidus absorbant les UV, comprenant l'incorporation dans un polymère de résidus dérivés d'un méthacrylate de méthyle et de résidus dérivés du composé tel que défini dans la revendication 1, dans lequel R est -CO.CR¹=CH₂ et R¹ est tel que défini dans la revendication 1.

6. Procédé fournissant un composé absorbant les UV, comprenant un procédé suivant la revendication 4 ou la revendication 5 dans lequel la quantité du composé de formule (I) est comprise entre 0,1 à 5 % en poids du polymère ou de la composition.

7. Article produit à partir d'un procédé suivant l'une quelconque des revendications 4 à 6.

8. Lentille intraoculaire ou lentille de contact comprenant un polymère produit selon un procédé suivant la revendication 5.

9. Lentille intraoculaire suivant la revendication 8, ayant au moins un haptique attaché à celle-ci ou formé de façon intégrale avec celle-ci.

10. Lentille suivant la revendication 9, ayant deux haptiques.

11. Article suivant la revendication 7, sous la forme de verres d'oeil ayant des lentilles faites à partir d'une composition de polymère comprenant une résine polycarbonate dans laquelle est incorporée une quantité absorbant les UV du composé de formule (I).
